# EUROPEAN PATENT APPLICATION

(11) **EP 1 661 559 A1**
(43) Date of publication of application: **31.05.2006**
(21) Application number: 04027997.8
(22) Date of filing: 25.11.2004
(51) Int. Cl.: A61K 9/51

(54) **Delivery vehicle manufactured by the miniemulsion method**

(71) Applicant: NanoDel Technologies GmbH, 39116 Magdeburg (DE)
(72) Inventor: Ringe, Kerstin Dr., 39122 Magdeburg (DE); Radunz, Hans-Eckart Prof-Dr., 64367 Mühltal (DE)
(74) Representative: Sandmann, Wolfgang

(57) **Abstract**

The present invention is directed to a method of producing a delivery vehicle by the miniemulsion method, said delivery vehicle at least comprising nanoparticles made by the miniemulsion method, a surface modifying agent and a pharmaceutical agent. The present invention is further directed to a delivery vehicle manufactured by this method and its use for the transfection of eucaryotic cells or for the treatment of diseases and conditions, requiring a pharmaceutical agent to cross one or more physiological barriers, in particular the blood-brain barrier.

## Description

The present invention is directed to a method of producing a delivery vehicle by the miniemulsion method, said delivery vehicle at least comprising nanoparticles made by the miniemulsion method, a surface modifying agent and a pharmaceutical agent. The present invention is further directed to a delivery vehicle manufactured by this method and its use for the transfection of eucaryotic cells or for the treatment of diseases and conditions, requiring a pharmaceutical agent to cross one or more physiological barriers, in particular the blood-brain barrier.

According to the prior art, nanoparticles may be prepared by what is generically termed as an "insolvent emulsification-evaporation technique" using single (oil-in-water) or multiple emulsifications (water-in-oil-in-water) depending upon whether the incorporated pharmaceutical agent is hydrophobic or hydrophilic.

After forming the nanoparticles, the solvent is evaporated from the emulsion. The nanoparticles are separated from the remainder by centrifugation, or preferably ultracentrifugation (120,000 to 145,000 g), washed with water, and re-centrifuged and decanted.

The conventional method of emulsion polymerisation is shortly explained in chapter Examples (see comparative Example).

From the prior art it is also known to carry out conversions to polymers in miniemulsions. Miniemulsions are dispersions of, e.g., water, an oil phase, and one or more surfactants which have a droplet size of from about 50 to 500 nm. The miniemulsions were considered metastable (cf. Emulsion Polymerization and Emulsion Polymers, Editors P. A. Lovell and Mohamed S. El-Aasser, John Wiley and Sons, Chichester, New York, Weinheim, 1997, pages 700 et seq.; Mohamed S. El-Aasser, Advances in Emulsion Polymerization and Latex Technology, 30^{th} Annual Short Course, Volume 3, Jun. 7-11, 1999, Emulsion Polymers Institute, Lehigh University, Bethlehem, Pa., USA). These dispersions find broad application in the art in cleaning products, cosmetics or body care products.

The preparation of aqueous primary dispersions by means of the free-radical miniemulsion polymerization of olefinically unsaturated monomers is known for example from International Patent Application WO 98/02466 or from German Patents DE-A-196 28 143 and DE-A-196 28 142. In the case of these known processes the monomers can be copolymerized in the presence of different low molecular mass, oligomeric or polymeric hydrophobic substances.

A preferred method of nanoparticle formation is the miniemulsion technique as developed by K. Landfester et al:

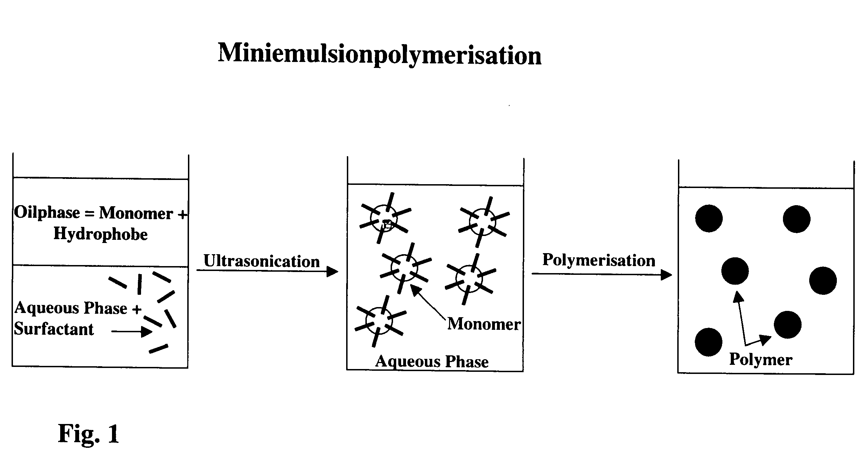

Here, miniemulsions are defined as dispersions of critically stabilized oil droplets with a size between 50 and 500 nm prepared by shearing a system containing water, a surfactant (or "stabilizer") and a hydrophobe. Polymerizations of monomers in such miniemulsions result in particles which have about the same size as the initial droplets. The principle of miniemulsion polymerization is schematically shown in Figure 1 (K. Landfester, Polyreactions in miniemulsions, Macromol. Rapid Comm. 2001, 896-936. K. Landfester, N. Bechthold, F. Tiarks, and M. Antonietti, Formulation and stability mechanisms of polymerizable miniemulsions. Macromolecules **1999,** *32,* 5222. K. Landfester, Recent Developments in Miniemulsions - Formation and Stability Mechanisms. Macromol. Symp. **2000,** *150,* 171).

S. C. Yang, H. X, Ge, Y. Hu, X. Q. Jiang, C. Z. Yang, describe in "Formation of positively charged polycyanacrylate nanoparticles stabilized with chitosan", Colloid. Polym. Sci., 2000, 278, 285-292, to design nanoparticles based on PBCA, containing a pharmaceutical agent (nimodipine) and a polysaccharide (chitosan). However, in this document, the nanoparticles are manufactured by emulsion polymerization and all ingredients are combined *ab initio,* i.e. before the polymerization process is started. Additionally, chitosan is incorporated in said nanoparticles and is not coated thereon. Furthermore, the process disclosed in this document involves the use of aceton as a solvent which is highly undesirable taking an intended *in vivo* use into consideration.

E. Marie, K. Landfester, M. Antonetti disclose in "Synthesis of chitosan-stabilized polymer dispersions", Biomacromolecules 2002, 3, 475-481, to manufacture styrene based nanoparticles having chitosan as a stabilizer integrated therein. However, this document mentions that the use of chitosan as a stabilizer alone is not advantageous, since that provides a certain degree of stiffness to the nanoparticles. This is avoided by adding biocompatible costabilizers with higher flexibility.

WO0209862 (DE 10101892) discloses a method for producing polymer capsules, pellets or droplets containing an active ingredient or substance, by means of in-situ encapsulation of said active ingredient or substance using a non-radical miniemulsion polymerisation, preferably polyaddition, of suitable monomers. The polymer vehicle system containing the active ingredient or substance, produced by the above method, can be used as a delivery system, in particular, in the field of cosmetics and bodycare, in pharmaceuticals, in adhesive processing and/or in the field of washing and cleaning agents.

DE19852784 discloses a process for stabilizing oil/water micro- or mini-emulsions with the aid of a surfactant (S1) and a co-surfactant (S2), in which (S2) consists of water-insoluble compound(s) selected to give an osmotically stabilized emulsion, other than hexadecane, cetyl alcohol, dodecylmercaptan, long-chain alkyl methacrylates and the dyestuff Blue 70. It further discloses osmotically stabilized micro- or mini-emulsions containing an oil phase, an aqueous phase, a surfactant (S1) and an ultra-hydrophobic co-surfactant (S2) as defined above.

WO2004017945 discloses the use of nanoparticles for a transfection of DNA into eucaryotic cells. It further discloses the DNA administration to a target organ in the human or animal body. In particular, WO2004017945 teaches the use of nanoparticles for the administration of cancer treatment-related DNA to a tumor-affected target organ in the human or animal body as, for example, the brain in the case of brain tumors.

WO2004017945 also discloses to use substances ("stabilizers") which act as enhancers of the bond between the DNA and the nanoparticles. Among others, Diethylaminoethyl-(DEAE)-dextran and dextran 70.000 are listed as stabilizers.

However, the only way for manufacturing those nanoparticles according to WO2004017945 is by conventional emulsion polymerization, i.e. forming nanoparticles directly from a solution of the monomers in a solvent. Furthermore, a surface modifying agent is not being used for specifically attaching the pharmaceutical agent to the nanoparticles. Furthermore, the stabilizers used in WO2004017945 are described as being incorporated in the nanoparticles and it is not suggested to coat the nanoparticles with said stabilizers.

Therefore, it is an object of the present invention to provide an improved method for the manufacture of drug delivery vehicles, which vehicles can be obtained in high yield and solid content. Furthermore, it is an object of the present invention to provide a method for the manufacture of drug delivery vehicles, which is suitable for the large-scale production of said vehicles. It is an additional object of the invention to provide a drug delivery vehicle which is capable of reversibly attaching a pharmaceutical agent in desired and specific amounts. Furthermore, it is an object of the invention to provide a delivery vehicle for a pharmaceutical agent which can be specifically adapted to desired release characteristics. It is in particular an object of the invention to provide a delivery vehicle which is suitable to be used for varying medical conditions and which is able to specifically cross the major physiological or biological barriers of the animal or human body and which subsequently is showing modified release characteristics such as sustained release or prolonged release of a pharmaceutical agent in the target tissue. It is an additional object of the present invention to avoid the use of stabilizers as, for example, chitosan, being incorporated into the nanoparticles which could confer negative structural characteristics to the nanoparticles.

These objects are achieved by the subject-matter of the independent claims. Preferred embodiments are set forth in the dependent claims.

As a general remark, the nanoparticles of the present invention are formed by a "miniemulsion method", which is per se well known and is, for example, disclosed in K. Landfester, "Polyreactions in miniemulsions", Macromol. Rapid Comm. 2001, 896-936. K. Landfester, N. Bechthold, F. Tiarks, and M. Antonietti, "Formulation and stability mechanisms of polymerizable miniemulsions". This technology basically differs from the well known conventional methods (as, for example, used in WO2004017945) by involving a different order of manufacturing steps: in this approach, a miniemulsion, containing dispersed hydrophobic, monomer containing droplets in a hydrophilic continous phase, are formed to polymeric particles. In the conventional approach, the polymers are directly formed from the solution containing monomers.

Thus, the term "miniemulsion" as used herein generally means a technology, by which a dispersion is made containing a continuous W (hydrophilic) and an O (hydrophobic) phase, dispersed in the continuous phase. Further, this term is directed to emulsion droplets formed of 1 to 1.000 nm, usually approximately between 50 and 500 nm (the size of the particles following polymerization is identical or almost identical).

One of the major drawbacks of the conventional approach can be seen in the fact that only suspensions containing about 1% of nanoparticles (solid content) may be obtained therewith. In contrast, the present miniemulsion approach allows a solid content of nanoparticles of even 15-24% or more based on a monomer content of 25% w/w (based on the overall weight of the O/W reaction system). Thus, the yield of solid nanoparticles is dramatically increased and corresponds to about 60-96% of the employed monomers.

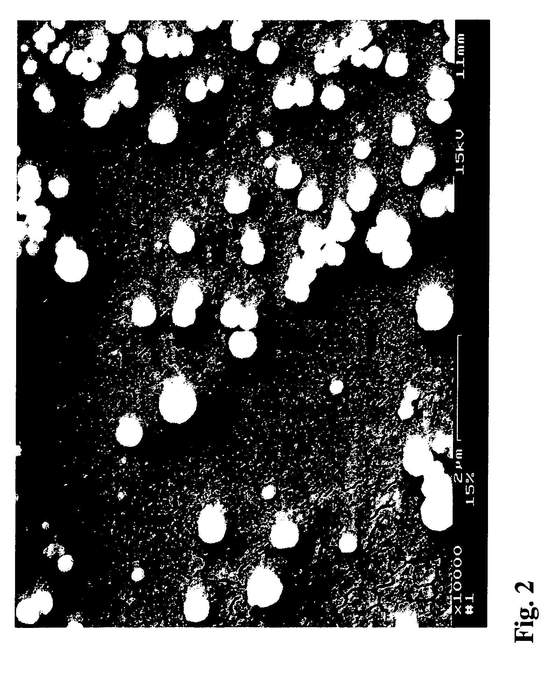

With this method, it is possible to obtain nanoparticle suspensions of high solid content (range: 15%-24%) and thus in a high yield (60-96%), which is very important in terms of up-scaling. The process is reproducible. The mean diameter of the generated nanoparticles is 200-300 nm (range < 1 µm) (see Figure 2).

As an example, if the O phase is consisting of 6 g monomer + 250 mg hydrophobe, the W phase is 24 g 0.01 N HCl + stabilizer, the maximum content of solids will be 25% w/w (6/24 x 100) corresponding to a yield of 100%. A content of 20% w/w then corresponds to a yield of 80%. In this case, the remaining 20% are represented by monomers, which were lost during polymerization, for example by polymerizing at the vessel surface or at the homogenizer or the like.

Furthermore, the approach of the present invention does not involve the incorporation of polysaccharide stabilizers, such as chitosan, into the nanoparticles. Thereby, negative influences of said materials on the physical properties of the nanoparticles (as increased stiffness or the like) as described in the prior art may be avoided.

A further, and very important drawback of the prior art approach of incorporating polysaccharides already in the inital phase of nanoparticle manufacturing, i.e. before the emulsion is formed and the polymerisation starts, resides in the fact that a major group of polysaccharides, dextrans, turned out not to be processible in the miniemulsion method used herein. Adding dextrans to the reaction system comprising O and W type liquid phases (which is required for manufacturing miniemulsions) did not led to any success, but instead, using the method of preparing a miniemulsion as disclosed in Landfester et al. (supra), only a foam-like substance was obtained.

Thus, the present invention is partially based on the surprising finding that polysaccharides as chitosan, dextran and the like are deleterious or at least disadvantageous for manufacturing nanoparticles by the miniemulsion method, if introduced in the reaction system in an early stage, i.e. before emulsification and polymerisation starts.

This disadvantage of the prior art approaches may be overcome by not incorporating polysaccharides, in particular dextrans or chitosan, into the nanoparticles and to avoid their use in an early stage of the miniemulsification process.

Instead, the present invention provides a coating of said polysaccharides (or of compounds derived therefrom) following the formation of the miniemulsion droplets or following their polymerisation. Therefore, a crucial step of the present method may be seen in the fact that those polysaccharides are not added to the reaction system before the preparation of the miniemulsion is completed.

It further unexpectedly turned out that the approach of the present invention, i.e. adding a coating of polysaccharide based surface modifying agents to the nanoparticles formed, may be used to precisely set the amount of a pharmaceutical agent bound thereon.

The chemical group of polysaccharides is in particular suitable to reach this object, since those substances are capable of binding pharmaceutical agents due to their polarity or charge (pH dependent) and are therefore providing an excellent means for reversibly binding pharmaceutical agents to nanoparticles in a precise amount. This effect is in particular important for pharmaceutical agents having a narrow therapeutic index in order to avoid delivery of said pharmaceutical agent in a too small (i.e. uneffective) or too large (i.e. toxic) amount to the tissue in question.

A further advantage of the present method of manufacturing pharmaceutical agents containing delivery vehicles is that pharmaceutical agents may be used, which usually would undergo destruction due to the high shear forces in the miniemulsion method. For example, it turned out that DNA as a pharmaceutical agent is mostly desintegrated during shearing the reaction system used for the preparation of miniemulsions. The present invention, however, offers for the first time the possibility of providing nanoparticles carrying highly unstable pharmaceutical agents, which nanoparticles being produced by the miniemulsion method.

### The present invention in particular is directed to the following aspects and embodiments:

According to a first aspect, the present invention is directed to a method of producing a delivery vehicle containing one or more pharmaceutical agents comprising the steps of:
a) providing a reaction system comprising O and W type liquid phases, a stabilizer and polymerizable monomers,
b) forming an O/W type miniemulsion, the O-phase droplets containing said monomers,
c) polymerizing said monomers in order to form nanoparticles,
d) adding a polysaccharide based surface modifying agent to said nanoparticles, and
e) adding one or more pharmaceutical agents capable of being bound via that surface modifying agent to said nanoparticles.

The term "reaction system" as used herein is defined as any environment, which fulfills the requirements of forming a miniemulsion according to the above definition.

As mentioned above, the already known miniemulsion technique as, for example, disclosed in Landfester et al. (supra), may be used to prepare the miniemulsion. This may, for example be done by at first combining monomers, an O and a W phase and a stabilizer (defined below). It is noted that the W phase may contain further ingredients, for example HCl for setting a suitable pH value. Second, the reaction system may be mixed by, for example, a homogenizer or the like, in order to mix all ingredients.

The preparation of the miniemulsion itself is performed by applying high shear forces to the reaction system, for example by ultrasound and high pressure homogenizers. Furthermore, the shear forces may be applied for a time range of from 2-5 min. depending on the size of the reaction system and the homogenizer used. Basically, a time range of between 3 and 4 min. is regarded as being sufficient.

The ultrasound homogenizer may have an amplitude of about 60-100%, preferably about 70%.

The amount of polysaccharide based surface modifying agent introduced into the reaction system may be set to between 0,1-50% w/w regarding the amount of monomeric substances used. The amount of 100% polysaccharide w/w regarding the amount of monomeric substances corresponds to those amounts, which are required in the conventional process of producing nanoparticles (other than miniemulsion). Therefore, by the method of the present invention, the high amounts of polysaccharides used in the prior art may be avoided, thus circumventing the problems, which are associated with their use (see above).

According to an embodiment, the temperatures used in this process are preferably from -1 to 5 °C, preferably 0°C.

Generally the weight ratio of O to W phase is from 15-40 % w/w, preferably 20-30 % w/w and more preferably about 25 % w/w.

Following preparing the miniemulsion (and, as one alternative, adding already in this stage the surface modifying agent), the polymerisation process is started, for example by simply increasing the pH to a value of pH 7,0 or the like. This may be done, whenever PBCA monomers are used in the reaction system and the pH increase is performed, for example, by adding a sufficient amount of K₂CO₃ to the reaction system.

The step of adding the surface modifying agent may, as a second alternative, also be performed prior to the polymerisation step. Therefore, according to a further embodiment, step d) is performed prior to step c).

The nanoparticles of the invention preferably have a diameter in the range of between 50 and 500 nm, more preferably between 200 and 300 nm (see Fig. 2 for an example).

According to a preferred embodiment, a stabilizer coating is applied to said nanoparticles. It is noted that the term "stabilizer" as used herein, is defined as any substance, which is maintaining an emulsion more stable, i.e. is a surfactant or the like. Without a stabilizer, a miniemulsion can hardly be achieved. It should be clear that in order to obtain an O/W type emulsion, the stabilizer must also have O/W forming characteristics. A treatment of the nanoparticles with a sufficient coating of an appropriate stabilizer allows the adsorbed pharmaceutical agent to better traverse physiological barriers as the bbb (= blood brain barrier). Reference is made to several documents disclosing this effect, in particular to WO 95/22963, which is incorporated herein in its entirety.

Preferably, after adsorbtion of the drug, the nanoparticles are coated with polysorbate 80 that allows the passage through the blood brain barrier.

In this reference, a novel method of delivering drugs and diagnostics across the blood-brain barrier or blood-nerve barrier is disclosed. Drugs or diagnostic agents are incorporated into nanoparticles which have been fabricated in conventional ways. These nanoparticles are then coated with additional surfactant and given to the body of animals or humans. This allows drugs or diagnostic agents to cross the blood-brain barrier (bbb) to achieve one or more of the following benefits: (1) reducing the dose of a therapeutic drug or diagnostic agent which, when given peripherally, maintains the biological or diagnostic potency in the nervous system, (2) allowing drugs that normally do not cross the bbb to penetrate into the nervous system, and (3) reducing the peripheral side effects by increasing the relative amount of the drug reaching the brain.

According to a preferred embodiment, the stabilizer comprises one or more of the following substances:
fatty acid esters of glycerols, sorbitol and other multifunctional alcohols, preferably, glycerol monostearate, sorbitan monolaurate, or sorbitan monoleate; poloxamines, preferably poloxamine 904 or 1508; polyoxyethylene ethers and polyoxyethylene esters; ethoxylated triglycerides; ethoxylated phenols and ethoxylated diphenols; surfactants of the Genapol TM and Bauki series; metal salts of fatty acids, metal salts of fatty alcohol sulfates, sodium lauryl sulfate; and metal salts of sulfosuccinates; preferably polysorbates, more preferably polysorbate 20, 40, 60 and most preferably polysorbate 80; preferably poloxamers, more preferably poloxamer 188, 338 or 407; preferably polyoxyethylene glycols, more preferably Lutensol 50 or 80; and mixtures of two or more of said substances.

According to a further embodiment, the O phase is containing a hydrophobe, preferably selected from olive oil, miglyol and/or hexadecane. The amount of hydrophobe is usually relatively small and should be sufficient to prevent Ostwald ripening (about 2-10% w/w based on the overall weight of the O-phase (further containing the monomer)).

According to a further embodiment, the polymeric materials used in the present method are selected from the group consisting of polyacrylates, polymethacrylates, polyalkylcyanoacrylates, polyalkylcyanacrylates, polyarylamides, polylactates, polyglycolates, polyanhydrates, polyorthoesters, gelatin, polysaccharides, albumin, polystyrenes, polyvinyls, polyacrolein, polyglutaraldehydes and derivatives, copolymers and mixtures thereof.

Preferred synthetic biodegradable polymeric materials comprise solid or film forming polymers, preferably polyalkylcyanoacrylates, more preferably polybutylcyanoacrylates, polylactic acid and polybutyric acid and mixtures and derivatives thereof.

As used herein, the terms "biodegradable polymer" denotes any synthetic or naturally-derived polymeric material which is known as being suitable for uses in the body of a living being, i.e., is biologically inert and physiologically acceptable, non-toxic, and, in the delivery systems of the present invention, is biodegradable in the environment of use, i.e., can be resorbed by the body.

Further biodegradable polymers which may be also used to formulate nanoparticles include, but are not limited to, polyesters, such as polyglycolides and polylactic polyglycolic copolymers (PLGA); polyethers, such as such as hydroxy-terminated poly(ε-caprolactone)-polyether or polycaprolactone (PCL); polyanhydrides; polyacrylamides; poly(orthoesters); polyamino acids; and biodegradable polyurethanes. It is to be understood that the term polymer is to be construed as to include copolymers and oligomers.

The term "pharmaceutical agents" as used herein, thus, comprises both, therapeutic agents and diagnostic agents. It is noted that the terms "drug" and "therapeutic agent" are used interchangeably herein.

The therapeutic agent is preferably selected from substances which are incapable of crossing physiological barrieres without a delivery vehicle or carrier. This physiological barrier is preferably selected from, although not restricted to, the group consisting of blood-brain barrier (bbb), blood-air barrier, blood-cerebrospinal fluid barrier and buccal mucosa. Additionally, the one or more therapeutic agents may have central nervous system activity but cannot cross the blood brain barrier without a delivery vehicle.

According to a preferred embodiment, the delivery vehicle of the invention comprises one or more therapeutic agents selected from the group consisting of drugs acting at synaptic sites and neuroeffector junctional sites; general and local analgetics; hypnotics and sedatives; drugs for the treatment of psychiatric disorders such as depression and schizophrenia; anti-epileptics and anticonvulsants; drugs for the treatment of Parkinson's and Huntington's disease, aging and Alzheimer's disease; excitatory amino acid antagonists, neurotrophic factors and neuroregenerative agents; trophic factors; drugs aimed at the treatment of CNS trauma or stroke; drugs for the treatment of addiction and drug abuse; antacoids and anti-inflammatory drugs; chemotherapeutic agents for parasitic infections and diseases caused by microbes; immunosuppressive agents and anti-cancer drugs; hormones and hormone antagonists; heavy metals and heavy metal antagonists; antagonists for non-metallic toxic agents; cytostatic agents for the treatment of cancer; diagnostic substances for use in nuclear medicine; immunoactive and immunoreactive agents; transmitters and their respective receptor agonists and receptor antagonists, their respective precursors and metabolites; transporter inhibitors; antibiotics; antispasmodics; antihistamines; antinauseants; relaxants; stimulants; sense and antisense oligonucleotides; cerebral dilators; psychotropics; antimanics; vascular dilators and constrictors; anti-hypertensives; drugs for migraine treatment; hypnotics, hyperglycemic and hypoglycemic agents; anti-asthmatics; antiviral agents, preferably anti HIV agents; genetic material suitable for the DNA or anti-sense treatment of diseases; and mixtures thereof.

The term "DNA"as used in the present specification basically refers to any DNA conceivable in the present field of the art. In preferred embodiments, the term "DNA" is meant to comprise two types of DNA, i. e. plasmid DNA, more preferably plasmid DNA comprising the information of tumor suppressor genes, even more preferably plasmid DNA comprising the information of the tumor suppressor genes p53 and pRB, on the one hand, and antisense oligonucleotides, more preferably antisense oligonucleotides against oncogenes, even more preferably antisense oligonucleotides against oncogenes like Bcl2, on the other hand. There may be used one type of DNA (and, consequently, one type of DNA-loaded nanoparticles) in the present invention. Alternatively, two or more types of DNA may be used, resulting into a plurality of types of nanoparticles loaded with different types of DNA and useable in accordance with the present invention.

Surprisingly, DNA and particularly DNA of the above two types could be adsorbed onto nanoparticles, and the resulting DNA-nanoparticle complexes could be inoculated into the organism, particularly into the organism suffering from cancer (specifically, but not limited to, brain cancer). Thereafter, a suppression of the tumor proliferation could be observed, and even a tumor necrosis and apoptosis could be induced.

In a preferred, but not essential embodiment of the invention, plasmid DNA comprising a promoter, more preferably plasmid DNA comprising an inducible promoter, can be loaded onto the nanoparticles. By the novel step to load the DNA containing an inducible promoter onto the nanoparticle and to express it within the cell, an inducible promoter, and thereby an external control of the expression of the relevant gene may be achieved, and the gene may be "switched" on and off at will. As an unexpected advantage over the prior art, the timing of the gene/DNA expression can be controlled. Such a control may reduce toxic side effects of a continuous gene expression and/or may lower the probability that cells become resistant to the gene products, producing a negative selection.

In a preferred embodiment of the invention, the human papilloma virus upstream regulatory region (HPV-URR) was used as the inducible promoter. The expression of the tumor suppressor is induced after the administration of Dexamethasone or other inducers or compounds. In that way, an apoptosis of the tumor cells as well as a regression of the tumor could be achieved. Other exemplary promoters useable in accordance with the present invention are the cytomegalia virus (CMV) promoter or the simian virus 40 (SV 40) promoter.

Furthermore, strong effects including a tumor regression could be achieved by a combination administration of one or more than one type(s) of DNA-containing nanoparticles and nanoparticles containing one or more than one cytostatically effective substance(s).

In a preferred embodiment, tumor suppressor DNA, even more preferred behind an inducible promoter, may be injected prior to inoculation of a nanoparticle complex containing a cytostatically effective compound. In an even more preferred embodiment, the cytostatically effective compound is Doxorubicine.

In the process of transfection of DNA into cells, and in the process of administration of a DNA to a target organ in the human or animal body as well, the first step comprises the preparation of nanoparticles in a way defined above.

For further information it is explicitely referred to WO 2004/017945, and its content is incorporated herein in its entirety.

Further typical active ingredients (e.g., drugs) can be any substance affecting the nervous system or used for diagnostic tests of the nervous system. These are described by Gilman *et al*. (1990), "Goodman and Gilman's - The Pharmacological Basis of Therapeutics", Pergamon Press, New York, and include the following agents:
acetylcholine and synthetic choline esters, naturally occurring cholinomimetic alkaloids and their synthetic congeners, anticholinesterase agents, ganglionic stimulants, atropine, scopolamine and related antimuscarinic drugs, catecholamines and sympathomimetic drugs, such as epinephrine, norepinephrine and dopamine, adrenergic agonists, adrenergic receptor antagonists, transmitters such as GABA, glycine, glutamate, acetylcholine, dopamine, 5-hydroxytryptamine, and histamine, neuroactive peptides; analgesics and anesthetics such as opioid analgesics and antagonists; preanesthetic and anesthetic medications such as benzodiazepines, barbiturates, antihistamines, phenothiazines and butylphenones; opioids; antiemetics; anticholinergic drugs such as atropine, scopolamine or glycopyrrolate; cocaine; chloral derivatives; ethchlorvynol; glutethimide; methyprylon; meprobamate; paraldehyde; disulfiram; morphine, fentanyl and naloxone; psychiatric drugs such as phenothiazines, thioxanthenes and other heterocyclic compounds (e.g., halperiodol); tricyclic antidepressants such as desimipramine and imipramine; atypical antidepressants (e.g., fluoxetine and trazodone), monoamine oxidase inhibitors such as isocarboxazid; lithium salts; anxiolytics such as chlordiazepoxyd and diazepam; anti-epileptics including hydantoins, anticonvulsant barbiturates, iminostilbines (such as carbamazepine), succinimides, valproic acid, oxazolidinediones and benzodiazepines. Anti-Parkinson drugs such as L-DOPA/ CARBIDOPA, apomorphine, amatadine, ergolines, selegeline, ropinorole, bromocriptine mesylate and anticholinergic agents;
antispasticity agents such as baclofen, diazepam and dantrolene; neuroprotective agents, such as excitatory amino acid antagonists, neurotrophic factors and brain derived neurotrophic factor, ciliary neurotrophic factor, or nerve growth factor; neurotrophine (NT) 3 (NT3); NT4 and NT5; gangliosides; neuroregenerative agents; drugs for the treatment of addiction and drug abuse include opioid antagonists and anti-depressants; autocoids and anti-inflammatory drugs such as histamine, bradykinin, kailidin and their respective agonists and antagonists; chemotherapeutic agents for parasitic infections and microbial diseases; anti-cancer drugs including alkylating agents (e.g., nitrosoureas) and antimetabolites; nitrogen mustards, ethylenamines and methylmelamines; alkylsulfonates; folic acid analogs; pyrimidine analogs, purine analogs, vinca alkaloids; and antibiotics.

The only requirement for all these substances is that they are present in a form capable of binding to the polysaccharide based surface modifying agent (examples will be explained below). For example, a apolar substance may be present in its salt form in order to bind to the surface modifying agent. If, for example, alginate is used as such a modifying agent (carrying negatively charged groups), an drug like morphine might be converted to morphine-HCl and then be bound to said alginate.

According to a preferred embodiment, the diagnostic agent is selected from the group consisting of diagnostics useful in the diagnosis in nuclear medicine and in radiation therapy.

The polysaccharide based surface modifying agents are preferably selected from dextrans, alginates, chitosan, or derivatives thereof.

The group of dextrans, as defined herein, comprises in particular: Dextran 12.000, Dextran 70.000 and Diethylaminoethyl-Dextran (DEAE-Dextran).

Chitosan is a natural polymer obtained by the hydrolysis of chitin, a native polymer present in shellfish. Together with chitin, chitosan is considered the second most abundant polysaccharide after cellulose. However, unlike cellulose, the use of chitosan as an excipient in pharmaceutical formulations is a relatively new development. Chitosan (poly[-(1,4)-2-amino-2-deoxy-D-glucopyranose]) has the following structure:

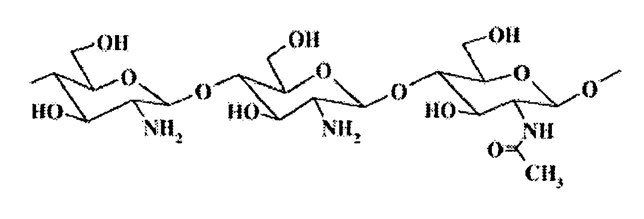

In an acidic pH (of, e.g. 5,5), the amino groups are carrying a positive charge.

The polymer differs from chitin in that a majority of the N-acetyl groups in chitosan are hydrolyzed (deacetylated). The degree of deacetylation has a significant effect on the solubility and rheological properties of the polymer. The amine group on the polymer has a pKa in the range of 5.5 to 6.5, depending on the source of the polymer. At low pH, the polymer is soluble, with the sol-gel transition occurring at approximate pH 7. The pH sensitivity, coupled with the reactivity of the primary amine groups, make chitosan a unique polymer for oral drug delivery applications.

Many derivatives of chitosan are available. The following are presented as examples: N-Trimethylene Chloride Chitosan (TMC), chitosan esters and chitosan conjugates.

Alginates are cell-wall constituents of brown algae (Phaeophycota). They are chain-forming heteropolysaccharides made up of blocks of mannuronic acid and guluronic acid. The composition of the blocks depends on the species being used for extraction and the part of the thallus from which extraction is made. Extraction procedures also affect alginate quality. Alginates usually are usually employed in this invention at a pH of 6-7.

Then, a negatively charged surface (Alginat) of nanoparticles leads to the binding of, preferential cationic drugs, that possess positively charged groups, such as dopamine.

In contrast, a positively charged surface (Chitosan, DEAE-Dextran, Cetylammonium bromide) of nanoparticles leads to the binding of, preferential DNA or oligonucleotides, because it possesses negatively charged phosphate groups.

Examples for drugs, which may be bound to alginates (apart from dopamine) are drugs, which are carrying amino function (-NH2, -NHR oder -NR2). Alkaloids, as morphine or naxolone; tricyclic antidepressants, as doxepine or trimipramine are very suitable, as well as drugs as diazepam, noradrenaline, phentolamine or phenylethylamine.

According to a preferred embodiment, the therapeutic agent and the surface modifying agent are selected from
a) a polysaccharide based modifying agent providing a positive or negative charge to the surface of the nanoparticles and a therapeutic agent which is negatively or positively charged, in order to provide a ionic binding between the modifying agent and the therapeutic agent, and wherein the modifying agent preferably is chitosan and the therapeutic agent preferably is genetic material suitable for the DNA or anti-sense treatment of diseases, or wherein the modifying agent preferably is alginate and the therapeutic agent preferably is dopamine; or
b) a polysaccharide based modifying agent providing a polar surface to the nanoparticles and the therapeutic agent being polar in order to provide a dipole-dipole binding between the modifying agent and the therapeutic agent, wherein the modifying agent preferably is dextran and the therapeutic agent preferably is a polar agent.

The most preferred combinations to be used in the present method are: alginates - dopamine, chitosan and/or dextrans - nucleic acids.

Even more preferred, in the above combinations, PBCA is used as substance to generate nanoparticles.

According to a further aspect, a delivery vehicle for pharmaceutical agents for administration to a mammal or a eucaryotic cell is disclosed comprising:
a) nanoparticles made of a polymeric material;
b) a polysaccharide based surface modifying agent, which is coated on said nanoparticles; and
c) one or more pharmaceutical agents, which are coated on and bind to the surface modifying agent; and optionally
d) a stabilizer coating deposited thereon,
obtainable by the method disclosed hereinabove.

According to a further aspect, the invention is directed to a pharmaceutical composition containing a delivery vehicle as defined above and a pharmaceutically acceptable carrier.

In order to use the nanoparticles in a practical embodiment *in vivo,* they may be reconstituted into a suspension with normal saline or phosphate buffered aqueous solution at physiological pH and osmolarity.

Typically, the nanoparticles are present in the injectable suspension at a concentration ranging from 0.1 mg nanoparticles per ml suspending fluid to 100 mg nanoparticles per ml suspending fluid. 10 mg nanoparticles per ml is preferred. The amount of nanoparticles used will strongly depend on the amount of pharmaceutical agent contained in an individual nanoparticle and the skilled artisan or the physician in charge will be readily able to adapt the dosage of the nanoparticles to the specific circumstances.

Alternatively, the pharmaceutical composition may take other forms required to transfer the delivery vehicle of the invention to and across other physiological barriers, for example to and across the blood-air barrier. Then it may, for example have the form of an aerosol or the like in order to deliver the composition by inhalation to the barrier in question.

According to a fourth aspect, the invention provides the use of a drug delivery vehicle or a pharmaceutical composition as defined herein for the transfection of eucaryotic cells or (for the manufacture of a medicament) for the treatment of diseases and conditions, requiring a pharmaceutical agent to cross one or more physiological barrieres, in particular the blood-brain barrier.

In particular, the delivery vehicle will find application in the treatment of diseases related to the CNS. Furthermore, this includes the treatment of AIDS, since in many people with advanced AIDS - possibly a third of adults and half of all children - HIV also infiltrates and harms the brain, triggering HIV-associated dementia. The disorder is marked by poor concentration, decreased memory and slow thinking and movements. However, it is particularly hard to target the virus in the brain. Here, the present invention may open up new therapeutic successes by delivering anti-HIV agents to the brain.

All publications, patent applications, patents, and other references mentioned herein are incorporated by reference in their entirety. In case of conflict, the present specification, including definitions, will control. In addition, the materials, methods, and examples are illustrative only and not intended to be limiting.

The invention is now further illustrated by the accompanying drawings, in which:
Fig. 1 is an illustration showing the miniemulsion polymerzation technique;
Fig. 2 is a photograph of nanoparticles manufactured in accordance with the present invention;
Fig. 3 is a representation of the particle size distribution of nanoparticles manufactured in Example 1.

### Examples:

### Comparative Example: The Conventional Method of Emulsion Polymerisation (prior art)

Preparation of PBCA-Nanoparticles: A solution of 10 mg of a mixture of dextran 70.000 in 1 ml 0.01 N HCl is prepared. After this, 0.01 ml butylcyanoacrylate is added carefully, and stirring is continued for 4 h at 25°C to assure complete polymerisation of the monomer. After neutralization by addition of 1 ml 0.01 M sodium hydroxide solution, larger polymer aggregates are separated from nanoparticles by filtration. For purification the prepared nanoparticle suspension is then centrifuged and resuspended in Millipore filtered water (3 times).

### Example 1:

### Preparation of PBCA-dextran-nanoparticles loaded with Daunorubizin and coated with Tween 80

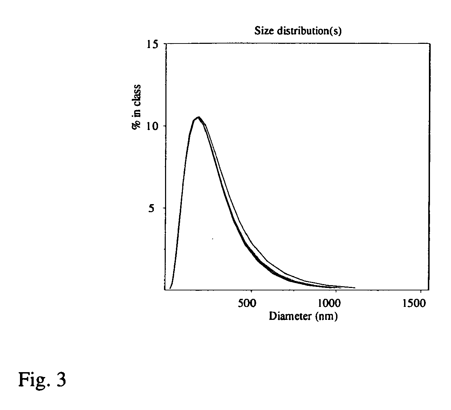

6 g of butylcyanacrylate and 250 ml mygliol were added to a 30 ml beaker. 500 mg Lutensol AT 80 were dissolved in 24 g 0.1 N HCl in another 30 ml beaker. Both solutions were combined and homogenzed for 2 min. using a dispersion stirrer. Then the miniemulsion was formed using ultrasound for 4 min. at an amplitude of 70% at 0°C. After the preparation of the miniemulsion 60 mg of Dextran were added and the pH was slowly (1 h) raised to 7 using a saturated K₂CO₃₋solution. The resulting nanoparticles were obtained as a suspension with a solid content of 15,5% (w/w) (155 mg/ml) and a particle size of 199 nm (see Fig. 3).

The obtained nanoparticle suspension was diluted to 1% (w/w) (10 mg/ml). 2.0 mg of Daunorubizin was added to 10 mg of the prepared nanoparticles. The resulting suspension was stirred for 4 h. To determine the loading rate the drug/nanoparticle suspension was centrifuged for 30 min. at 20.000 rpm. The supernatant was analyzed using a UV-spectrometer that was previously calibrated using different solutions of Daunorubizin at a known concentration. The loading rate could be determined as 45% (0.9 mg of Daunorubizin was bound to 10 mg of nanoparticles).

The pellet was then resuspended in a 1% (w/w) Tween® 80 solution containing 0.9% NaCl.

### Example 2: Preparation of PBCA-dextran-nanopardcles loaded with Mithramyzin and coated with Tween 80

6 g of butylcyanacrylate and 250 ml mygliol were added to a 30 ml beaker. 500 mg Lutensol AT 50 were dissolved in 24 g 0.1 N HCl in another 30 ml beaker. Both solutions were combined and homogenized for 2 min. using a dispersion stirrer. Then the miniemulsion was formed using ultrasound for 4 min. at an amplitude of 70% at 0°C. After the preparation of the miniemulsion 960 mg of Dextran were added and the pH was slowly (1 h) raised to 7 using a saturated K₂CO₃₋solution. The resulting nanoparticles were obtained as a suspension with a solid content of 24% (w/w) (240 mg/ml) and a particle size of 230 nm.

The obtained nanoparticle suspension was diluted to 1% (w/w) (10 mg/ml). 2.0 mg of Mithramyzin was added to 10 mg of the prepared nanoparticles. The resulting suspension was stirred for 4 h. To determine the loading rate the drug/nanoparticle suspension was centrifuged for 30 min. at 20000 rpm. The supernatant was analyzed using a UV-spectrometer that was previously calibrated using different solutions of Mithramyzin at a known concentration. The loading rate could be determined as 59% (1.18 mg of Mithramyzin was bound to 10 mg of nanoparticles).

The pellet was then resuspended in a 1% (w/w) Tween® 80 solution containing 0.9% NaCl.

### Example 3: Preparation of PBCA-dextran-nanoparticles loaded with Mithramyzin and coated with Tween 80

This example is illustrating on an embodiment, in which the surface modifying agent is added following the polymerzation step.

6 g of butylcyanacrylate and 250 ml mygliol were added to a 30 ml beaker. 500 mg Lutensol AT 50 are dissolved in 24 g 0.1 N HCl in another 30 ml beaker. Both solutions are combined and homogenized for 2 min. using a dispersion stirrer. Then the miniemulsion was formed using ultrasound for 4 min. at an amplitude of 70% at 0°C. After the preparation of the miniemulsion the pH was slowly (1 h) raised to 7 using a saturated K₂CO₃-solution. Then 960 mg of Dextran were added. The resulting nanoparticles were obtained as a suspension with a solid content of 24% (w/w) (240 mg/ml) and a particle size of 242 nm.

The obtained nanoparticle suspension was diluted to 1% (w/w) (10 mg/ml). 2.0 mg of Mithramyzin was added to 10 mg of the prepared nanoparticles. The resulting suspension was stirred for 4 h. To determine the loading rate the drug/nanoparticle suspension was centrifuged for 30 min. at 20.000 rpm. The supernatant was analyzed using a UV-spectrometer that was previously calibrated using different solutions of Mithramyzin at a known concentration. The loading rate could be determined as 58% (1.15 mg of Mithramyzin was bound to 10 mg of nanoparticles).

The pellet was then resuspended in a 1% (w/w) Tween® 80 solution containing 0.9% NaCl.

### Example 4: Preparation of PBCA-dextran-nanoparticles loaded with Mitomyzin and coated with Tween 80

6 g of butylcyanacrylate and 250 ml mygliol were added to a 30 ml beaker. 500 mg Lutensol AT 50 were dissolved in 24 g 0.1 N HCl in another 30 ml beaker. Both solutions were combined and homogenized for 2 min. using a dispersion stirrer. Then the miniemulsion was formed using ultrasound for 4 min. at an amplitude of 70% at 0°C. After the preparation of the miniemulsion 1.2 g of Dextran were added and the pH was slowly (1 h) raised to 7 using a saturated K₂CO₃₋solution. The resulting nanoparticles were obtained as a suspension with a solid content of 11.2% (w/w) (112 mg/ml) and a particle size of 205 nm.

The obtained nanoparticle suspension was diluted to 1% (w/w) (10 mg/ml). 2.5 mg of Mitomyzin was added to 10 mg of the prepared nanoparticles. The resulting suspension was stirred for 4 h. To determine the loading rate the drug/nanoparticle suspension was centrifuged for 30 min. at 20.000 rpm. The supernatant was analyzed using a UV-spectrometer that was previously calibrated using different solutions of Mitomyzin at a known concentration. The loading rate could be determined as 87% (2.17 mg of Mitomyzin was bound to 10 mg of nanoparticles).
The pellet was then resuspended in a 1% (w/w) Tween® 80 solution containing 0.9% NaCl.

### Example 5: Preparation of PBCA-dextran-nanoparticles loaded with Mitomyzin and coated with Tween 80

This example is illustrating on an embodiment, in which the surface modifying agent is added following the polymerzation step.

6 g of butylcyanacrylate and 250 ml mygliol were added to a 30 ml beaker. 500 mg Lutensol AT 50 are dissolved in 24 g 0.1 N HCl in another 30 ml beaker. Both solutions were combined and homogenized for 2 min. using a dispersion stirrer. Then the miniemulsion was formed using ultrasound for 4 min. at an amplitude of 70% at 0°C. After the preparation of the miniemulsion the pH is slowly (1 h) raised to 7 using a saturated K₂CO₃-solution. Then 1.2 g of Dextran were added. The resulting nanoparticles were obtained as a suspension with a solid content of 12.7% (w/w) (127 mg/ml) and a particle size of 171 nm.

The obtained nanoparticle suspension was diluted to 1% (w/w) (10 mg/ml). 2.5 mg of Mitomyzin was added to 10 mg of the prepared nanoparticles. The resulting suspension was stirred for 4 h. To determine the loading rate the drug/nanoparticle suspension was centrifuged for 30 min. at 20000 rpm. The supernatant was analyzed using a UV-spectrometer that was previously calibrated using different solutions of Mitomyzin at a known concentration. The loading rate could be determined as 87% (2.18 mg of Mitomyzin was bound to 10 mg of nanoparticles).

The pellet was then resuspended in a 1% (w/w) Tween® 80 solution containing 0.9% NaCl.

### Example 6: Preparation of PBCA-alginat-nanoparticles loaded with Dopamine and coated with Tween 80

6 g of butylcyanacrylate and 250 ml mygliol were added to a 30 ml beaker. 500 mg Lutensol AT 80 were dissolved in 24 g 0.1 N HCl in another 30 ml beaker. Both solutions were combined and homogenized for 2 min. using a dispersion stirrer. Then the miniemulsion was formed using ultrasound for 4 min. at an amplitude of 70% at 0°C. After the preparation of the miniemulsion 120 mg of Alginat were added and the pH was slowly (1 h) raised to 7 using a saturated K₂CO₃₋solution. The resulting nanoparticles were obtained as a suspension with a solid content of 17,2% (w/w) (172 mg/ml) and a particle size of 140 nm.

The obtained nanoparticle suspension was diluted to 1% (w/w) (10 mg/ml). 2.5 mg of Dopamine was added to 10 mg of the prepared nanoparticles. The resulting suspension was stirred for 4 h. To determine the loading rate the drug/nanoparticle suspension was centrifuged for 30 min. at 20.000 rpm. The supernatant was analyzed using a UV-spectrometer that was previously calibrated using different solutions of Dopamine at a known concentration. The loading rate could be determined as 55% (1.37 mg of Dopamine was bound to 10 mg of nanoparticles).

The pellet was then resuspended in a 1% (w/w) Tween® 80 solution containing 0.9% NaCl.

### Example 7: Preparation of PBCA-chitosan-nanoparticles

6 g of butylcyanacrylate and 250 ml mygliol were added to a 30 ml beaker. 500 mg Lutensol AT 80 were dissolved in 24 g 0.1 N HCl in another 30 ml beaker. Both solutions were combined and homogenized for 2 min. using a dispersion stirrer. Then the miniemulsion was formed using ultrasound for 4 min. at an amplitude of 70% at 0°C. After the preparation of the miniemulsion 120 mg of chitosan were added and the pH was slowly (1 h) raised to 7 using a saturated K₂CO₃₋solution. The resulting nanoparticles were obtained as a suspension with a solid content of 20% (w/w) (200 mg/ml) and a particle size of 273 nm.

## Claims

1. A method of producing a delivery vehicle containing one or more pharmaceutical agents, which comprises the steps of:
a) providing a reaction system comprising O and W type liquid phases, a stabilizer and polymerizable monomers,
b) forming an O/W type miniemulsion, the O-phase droplets containing said monomers,
c) polymerizing said monomers in order to form nanoparticles,
d) adding a polysaccharide based surface modifying agent to said nanoparticles, and
e) adding one or more pharmaceutical agents capable of being bound via that surface modifying agent to said nanoparticles.

2. The method of claim 1, wherein step d) is performed prior to step c).

3. The method of claim 1, wherein a stabilizer coating is applied to said nanoparticles.

4. The method of one or more of claims 1-3, wherein the O phase contains olive oil, miglyol and/or hexadecane.

5. The method of one or more of the preceding claims, wherein the polymeric material is selected from the group consisting of polyacrylates, polymethacrylates, polyalkylcyanoacrylates, polyarylamides, polylactates, polyglycolates, polyanhydrates, polyorthoesters, gelatin, polysaccharides, albumin, polystyrenes, polyvinyls, polyacrolein, polyglutaraldehydes and derivatives, copolymers and mixtures thereof.

6. The method of one or more of the preceding claims, wherein the stabilizer provided in step a) or applied in claim 3 comprises one or more of the following substances:
fatty acid esters of glycerols, sorbitol and other multifunctional alcohols, preferably, glycerol monostearate, sorbitan monolaurate, or sorbitan monoleate; poloxamines, preferably poloxamine 904 or 1508; polyoxyethylene ethers and polyoxyethylene esters; ethoxylated triglycerides; ethoxylated phenols and ethoxylated diphenols; surfactants of the Genapol TM and Bauki series; metal salts of fatty acids, metal salts of fatty alcohol sulfates, sodium lauryl sulfate; and metal salts of sulfosuccinates; preferably polysorbates, more preferably polysorbate 60 and most preferably polysorbate 80; preferably poloxamers, more preferably poloxamer 188, 338 or 407; preferably polyoxyethylene glycols, more preferably Lutensol 50 or 80; and mixtures of two or more of said substances.

7. The method of one or more of the preceding claims, wherein the one or more pharmaceutical agents are selected from therapeutic agents and diagnostic agents, wherein the therapeutic agent preferably is selected from therapeutic agents having central nervous system activity but cannot cross the blood brain barrier without a delivery vehicle, and wherein the diagnostic agent is selected from the group consisting of diagnostics useful in the diagnosis in nuclear medicine and in radiation therapy.

8. The method of one or more of the preceding claims, wherein the polysaccharides are selected from dextrans, alginates, chitosan, or derivatives thereof.

9. The method of one or more of the preceding claims, wherein the therapeutic agent and the surface modifying agent are selected from
a) a polysaccharide based modifying agent providing a positive or negative charge to the surface of the nanoparticles and a therapeutic agent which is negatively or positively charged, in order to provide a ionic binding between the modifying agent and the therapeutic agent, and wherein the modifying agent preferably is chitosan and the therapeutic agent preferably is genetic material suitable for the DNA or anti-sense treatment of diseases, or wherein the modifying agent preferably is alginate and the therapeutic agent preferably is dopamine; or
b) a polysaccharide based modifying agent providing a polar surface to the nanoparticles and the therapeutic agent being polar in order to provide a dipole-dipole binding between the modifying agent and the therapeutic agent, wherein the modifying agent preferably is dextran and the therapeutic agent preferably is a polar agent.

10. A delivery vehicle containing pharmaceutical agents for administration to a mammal or a eucaryotic cell comprising:
a) nanoparticles made of a polymeric material;
b) a polysaccharide based surface modifying agent, which is coated on said nanoparticles; and
c) one or more pharmaceutical agents, which are coated on and bind to the surface modifying agent; and optionally
d) a stabilizer coating deposited thereon,
obtainable by the method of one or more of claims 1-9.

11. A pharmaceutical composition containing a delivery vehicle of claim 10 and a pharmaceutically acceptable carrier.

12. Use of a drug delivery vehicle of claim 10 or a pharmaceutical composition of claim 11 for the transfection of eucaryotic cells or for the manufacture of a medicament for the treatment of diseases and conditions, requiring a pharmaceutical agent to cross one or more physiological barrieres, in particular the blood-brain barrier.
